# EUROPÄISCHE PATENTANMELDUNG

(11) **EP 3 299 863 A1**
(43) Veröffentlichungstag der Anmeldung: **28.03.2018**
(21) Anmeldenummer: 17189239.1
(22) Anmeldetag: 04.09.2017
(51) Int. Cl.: G02B 23/24

(54) **ENDOSKOP UND VERFAHREN ZUM ZENTRIEREN EINES OKULARS IN EINEM HAUPTTEIL EINES ENDOSKOPS**

(30) Priorität: 21.09.2016 DE 102016117805
(71) Anmelder: Henke-Sass, Wolf GmbH, 78532 Tuttlingen (DE)
(72) Erfinder: WAIZENEGGER, Markus, 78570 Mühlheim (DE); SEEH, Daniel, 78194 Immendingen (DE)
(74) Vertreter: Patentanwälte Geyer, Fehners & Partner mbB

(57) **Zusammenfassung**

Die Erfindung schafft ein Endoskop (10), umfassend ein Hauptteil (14), welches eine Zentrierbuchse (32) mit einer Innenfläche (46), die eine Rotationsachse (RA) definiert, umfasst; eine erste Exzenterbuchse (34), welche in der Zentrierbuchse (32) um die Rotationsachse (RA) drehbar gelagert ist und eine erste Zentrierinnenfläche (56) aufweist, die eine erste Drehachse (D1) definiert, wobei die erste Drehachse (D1) zu der Rotationsachse (RA) um einen ersten Versatz (58) parallel versetzt ist; eine zweite Exzenterbuchse (36), welche in der ersten Exzenterbuchse (34) um die erste Drehachse (D1) drehbar gelagert ist und eine zweite Zentrierinnenfläche (68) aufweist, die eine zweite Drehachse (D2) definiert, wobei die zweite Drehachse (D2) zu der ersten Drehachse (D1) um einen zweiten Versatz (70) parallel versetzt ist; und ein Okular (24), welches mit der zweiten Exzenterbuchse (36) verbunden ist und eine optische Achse aufweist, die parallel zu der zweiten Drehachse (D2) ist.

## Beschreibung

Die Erfindung betrifft ein Endoskop, welches ein Hauptteil und ein Okular umfasst. Ferner betrifft die Erfindung ein Verfahren zum Zentrieren eines Okulars in einem Hauptteil eines Endoskops.

Aus dem Stand der Technik bekannte Endoskope weisen ein Objektiv und ein Okular auf. Die von dem Objektiv gesammelte Strahlung wird über ein optisches Leitsystem dem Okular zugeführt, so dass ein Objekt oder eine Probe mit Hilfe des Endoskops betrachtet werden kann. Das optische Leitsystem kann aus mehreren Linsen aufgebaut sein, die zueinander zentriert werden. Das Objektiv, die Vielzahl von Linsen und das Okular haben jeweils eine optische Achse, die zueinander ausgerichtet werden müssen. Aufgrund von Fertigungstoleranzen ist ein Justierungssystem notwendig, um beispielsweise die optische Achse des Okulars hinsichtlich der optischen Achse der Linsen des Leitsystems einzustellen. Ferner kann es aufgrund von Temperaturschwankungen, beispielsweise beim Autoklavieren des Endoskops, dazu kommen, dass sich die optische Achse des Okulars gegenüber der optischen Achse des Objektivs oder der Linsen des Leitsystems verschiebt.

Im Stand der Technik wird dieses Justierungssystem durch drei Stellschrauben realisiert, welche am Mantel einer Buchse eines Hauptteils des Endoskops angeordnet sind. In der Buchse ist das Okular aufgenommen, so dass durch das Hinein- oder Herausdrehen der entsprechenden Stellschrauben die optische Achse des Okulars verschoben werden kann.

Aufgabe der Erfindung ist es, ein Endoskop bereitzustellen, bei dem die optische Achse des Okulars zuverlässig und schnell justiert werden kann.

Die Aufgabe wird durch das Endoskop nach Anspruch 1 sowie durch das Verfahren nach Anspruch 10 gelöst. Die abhängigen Ansprüche beschreiben bevorzugte Ausführungsformen der Erfindung.

Die Erfindung schafft ein Endoskop, welches ein Hauptteil, eine erste Exzenterbuchse, eine zweite Exzenterbuchse und ein Okular umfasst. Das Hauptteil weist ein proximales Ende und eine Zentrierbuchse an dem proximalen Ende auf. Die Zentrierbuchse umfasst eine Innenfläche, die eine Rotationsachse definiert. Die erste Exzenterbuchse ist in der Zentrierbuchse an dem proximalen Ende um die Rotationsachse drehbar gelagert und weist eine erste Zentrierinnenfläche auf, die eine erste Drehachse definiert, wobei die erste Drehachse zu der Rotationsachse um einen ersten Versatz parallel versetzt ist. Die zweite Exzenterbuchse ist in der ersten Exzenterbuchse um die erste Drehachse drehbar gelagert und weist eine zweite Zentrierinnenfläche auf, die eine zweite Drehachse definiert, wobei die zweite Drehachse zu der ersten Drehachse um einen zweiten Versatz parallel versetzt ist. Das Okular ist mit der zweiten Exzenterbuchse verbunden und weist eine optische Achse auf, die parallel zu der zweiten Drehachse ist. Das Okular ist durch Drehen der ersten Exzenterbuchse und/oder der zweiten Exzenterbuchse radial zu der Rotationsachse versetzbar.

Vorteil der Erfindung ist, dass das Zentrieren des Okulars, d.h. das Verschieben der optischen Achse des Okulars parallel zu der Rotationsachse, besonders einfach dadurch gelingt, dass die erste Exzenterbuchse und/oder die zweite Exzenterbuchse in Umfangsrichtung gedreht werden. Es sind somit keine Werkzeuge notwendig, wie dies beispielsweise bei dem Verstellen der Stellschrauben im Stand der Technik erforderlich ist. Darüber hinaus besteht bei der Verwendung der Stellschrauben aus dem Stand der Technik die Gefahr, dass das Okular verkippen kann, d.h. dass die optische Achse des Okulars einen Winkel gegenüber der optischen Achse der Linsen des Leitsystems oder der Rotationsachse des Hauptteils einschließt, was die Abbildung des Objekts beeinträchtigen kann. Dadurch dass die erste Exzenterbuchse in dem Hauptteil und die zweite Exzenterbuchse in der ersten Exzenterbuchse drehbar gelagert sind, kann es zu keinem Verkippen des Okulars beim Drehen der ersten und/oder zweiten Exzenterbuchse kommen. Vielmehr wird dadurch ermöglicht, dass es nur zu einem parallelen Versatz der optischen Achse des Okulars kommt. Ein Verkippen ist praktisch ausgeschlossen. Ein weiterer Vorteil ist, dass das Justieren des Okulars besonders einfach ist, da es lediglich zwei Parameter zum Verstellen gibt und nicht drei wie bei dem System der Stellschrauben. Hier muss lediglich die erste Exzenterbuchse und/oder die zweite Exzenterbuchse gedreht werden.

Das Justieren der optischen Achse des Okulars erfolgt optional dadurch, dass die erste Exzenterbuchse eine erste Zentrieraußenfläche und die zweite Exzenterbuchse eine zweite Zentrieraußenfläche aufweist. Die erste Zentrieraußenfläche liegt beispielsweise an der Innenfläche der Zentrierbuchse des Hauptteils an und ist dadurch formschlüssig drehbar gelagert, und zwar so, dass die erste Zentrieraußenfläche sich um die Rotationsachse der Zentrierbuchse dreht. Dadurch dass die erste Drehachse der ersten Zentrierinnenfläche der ersten Exzenterbuchse parallel um den ersten Versatz zur Rotationsachse versetzt ist, kann die Position der ersten Drehachse durch Drehen der ersten Exzenterbuchse verändert werden.

Dadurch dass die erste Exzenterbuchse um die Rotationsachse an der Innenfläche der Zentrierbuchse drehbar gelagert ist, kommt es insbesondere zu keinem Verkippen der ersten Drehachse gegenüber der Rotationsachse sondern lediglich zu einem parallelen Versatz. Der erste Versatz gibt den Abstand zwischen der Rotationsachse und der ersten Drehachse an. Durch Drehen der ersten Exzenterbuchse kann die Richtung des Versatzes geändert werden.

Da die zweite Exzenterbuchse, beispielsweise durch Anliegen der zweiten Zentrieraußenfläche an der ersten Zentrierinnenfläche, drehbar um die erste Drehachse in der ersten Exzenterbuchse gelagert ist, wird durch Drehen der zweiten Exzenterbuchse um die erste Drehachse die zweite Drehachse parallel zu der ersten Drehachse versetzt; der Grund dafür ist der zweite Versatz. Die Länge des zweiten Versatzes gibt den Abstand zwischen der ersten Drehachse und der zweiten Drehachse an. Durch Drehen der zweiten Exzenterbuchse kann die Richtung des zweiten Versatzes eingestellt werden. Dadurch dass das Okular mit der zweiten Exzenterbuchse verbunden ist und somit die optische Achse des Okulars fest gegenüber der zweiten Drehachse ist, lässt sich die optische Achse des Okulars in der radialen Richtung der zweiten Exzenterbuchse verschieben. Die optische Achse kann mit der zweiten Drehachse zusammenfallen.

Durch Drehen der ersten Exzenterbuchse lässt sich die optische Achse des Okulars um den ersten Versatz in eine erste Richtung parallel verschieben und durch Drehen der zweiten Exzenterbuchse lässt sich die optische Achse in eine zweite Richtung um den zweiten Versatz verschieben. Da die erste Exzenterbuchse und die zweite Exzenterbuchse vorzugsweise um 360° verdrehbar sind, lässt sich somit jede beliebige Richtung für den ersten und/oder zweiten Versatz einstellen. Die optische Achse des Okulars ist somit maximal um die Summe des ersten Versatzes und des zweiten Versatzes gegenüber der Rotationsachse verschiebbar. Dies ist dann der Fall, wenn der erste Versatz und der zweite Versatz in die gleiche Richtung zeigen. Innerhalb eines Kreises mit dem Radius gleich der Summe des ersten Versatzes und des zweiten Versatzes um die Rotationsachse kann die optische Achse durch Drehen der ersten Exzenterbuchse und der zweiten Exzenterbuchse nahezu jede Position einnehmen.

Der erste Versatz kann im mathematischen Sinne als ein erster Vektor verstanden werden, der von der Rotationsachse senkrecht absteht; er legt die radiale Richtung der ersten Exzenterbuchse fest. Der zweite Versatz kann in diesem Sinne als ein zweiter Vektor verstanden werden, der an der Spitze des ersten Vektors angeordnet ist. Dies ergibt sich dadurch, dass der zweite Vektor von der ersten Drehachse ausgeht, welche an der Spitze des ersten Vektors angeordnet ist. Durch Drehen der ersten und/oder zweiten Exzenterbuchse lässt sich die Richtung der beiden Vektoren verändern. Die zweite Drehachse, und optional die optische Achse, befindet sich an der Spitze des zweiten Vektors.

Das Endoskop kann wie ein aus dem Stand der Technik bekanntes Endoskop ausgebaut sein und weist optional neben dem Hauptteil einen Schaft auf, in welchem ein Objektiv und/oder Linsen eines optischen Leitsystems angeordnet sind. Das Objektiv ist insbesondere an einem distalen Ende des Endoskops angeordnet.

An dem proximalen Ende weist das Hauptteil die Zentrierbuchse auf, welche die erste Exzenterbuchse, die zweite Exzenterbuchse und das Okular stützt. Die Zentrierbuchse, die erste Exzenterbuchse, die zweite Exzenterbuchse und das Okular bilden zusammen eine Okularzentriervorrichtung zum Zentrieren des Okulars gegenüber dem Hauptteil. Die Zentrierbuchse weist eine Innenfläche auf, die beispielsweise im Querschnitt senkrecht zur Rotationsachse kreisförmig ist, wobei der Mittelpunkt des Kreises auf der Rotationsachse liegt. In einer anderen Ausführungsform ist die Innenfläche im Querschnitt senkrecht zur Rotationsachse aus Kreissegmenten aufgebaut, deren Radius einen Mittelpunkt aufweist, welcher auf der Rotationsachse liegt. Die Innenfläche definiert insbesondere eine Fläche, in der sich ein Zylinder um die Rotationsachse drehbar gelagert ist. In einer beispielhaften Weiterbildung ist die Innenfläche der Zentrierbuchse die Innenfläche eines Hohlzylinders, in welchem beispielsweise eine oder mehrere sich in Richtung der Rotationsachse erstreckende Spalte vorgesehen sind, wobei die Symmetrieachse des Hohlzylinders der Rotationsachse entspricht.

Die erste Exzenterbuchse ist vorzugsweise mindestens um die Länge des ersten Versatzes drehbar in der Zentrierbuchse des Hauptteils gelagert. Die drehbare Lagerung kann als Gleitlager oder Wälzlager ausgebildet sein. Die erste Exzenterbuchse hat, wie oben beschrieben, optional eine erste Zentrieraußenfläche, welche im Querschnitt senkrecht zu der Rotationsachse und/oder der ersten Drehachse kreisförmig ist und einen Mittelpunkt aufweist, der zu der ersten Drehachse um den ersten Versatz parallel versetzt ist. Die erste Zentrieraußenfläche kann auch im Querschnitt senkrecht zur Rotationsachse und/oder der ersten Drehachse aus mehreren Kreissegmenten aufgebaut sein, deren jeweiliger Mittelpunkt zu der ersten Drehachse parallel versetzt ist. Insbesondere liegt der Mittelpunkt der ersten Zentrieraußenfläche auf der Rotationsachse der Zentrierbuchse. Die erste Zentrieraußenfläche ist optional derart ausgestaltet, dass sie wie ein Zylinder in der Zentrierbuchse drehbar gelagert ist. Die Symmetrieachse dieses Zylinders stimmt mit der Rotationsachse überein und ist um den ersten Versatz zu der ersten Drehachse versetzt.

Die erste Zentrierinnenfläche kann analog zu der Innenfläche der Zentrierbuchse ausgestaltet sein; insbesondere gelten die im Zusammenhang mit der Innenfläche der Zentrierbuchse angestellten Überlegungen für die erste Zentrierinnenfläche analog. Die erste Zentrierinnenfläche kann auf gleicher axialer Höhe wie die erste Zentrieraußenfläche angeordnet sein oder zu dieser axial versetzt sein.

Die hinsichtlich der ersten Exzenterbuchse angestellten Überlegungen gelten vorzugsweise analog für die zweite Exzenterbuchse beschriebenen Varianten und Weiterbildungen. Optional ist ein Bereich der zweiten Exzenterbuchse, mittels welchem die zweite Exzenterbuchse in der ersten Exzenterbuchse drehbar gelagert ist, auf einer gleichen axialen Höhe wie ein Bereich der ersten Exzenterbuchse, mittels welchem die erste Exzenterbuchse in der Zentrierbuchse drehbar gelagert ist.

Die Zentrierbuchse, die erste Exzenterbuchse und/oder die zweite Exzenterbuchse können aus Metall, insbesondere Edelstahl, hergestellt sein. Die Rotationsachse, die erste Drehachse, die zweite Drehachse und/oder die optische Achse können parallel zueinander verlaufen.

Das Okular kann an der zweiten Exzenterbuchse dauerhaft fixiert sein oder mittels Befestigungsmittel an der zweiten Exzenterbuchse angebracht sein. Beispielsweise kann das Okular in einen von der zweiten Zentrierinnenfläche definierten Hohlraum hineingeschoben werden und anschließend an der zweiten Exzenterbuchse fixiert werden. Zum Justieren ist es notwendig, dass zumindest während des Justierens die optische Achse des Okulars in einem festen eingestellten Abstand gegenüber der zweiten Drehachse ist. Das Okular kann eine oder mehrere Linsen aufweisen, die beispielsweise zueinander verschiebbar sind, um die Abbildungseigenschaften des Okulars zu verändern.

Das Endoskop kann eine Fixiereinrichtung aufweisen, mittels welcher die relative Lage der ersten Exzenterbuchse zu der Zentrierbuchse, der zweiten Exzenterbuchse zu der ersten Exzenterbuchse und/oder das Okular gegenüber der zweiten Exzenterbuchse vor oder nach dem Justieren fixiert werden kann. Beispielsweise können Schrauben vorgesehen sein, die durch Anziehen eine Relativverdrehung der ersten Exzenterbuchse zu der Zentrierbuchse oder der zweiten Exzenterbuchse blockiert werden kann.

Die erste und/oder zweite Exzenterbuchse kann jeweils einen Zentrierabschnitt und einen Kragen aufweisen. Der Zentrierabschnitt weist optional die oben beschriebenen Eigenschaften auf, so dass mit dem jeweiligen Zentrierabschnitt der Versatz der optischen Achse gegenüber der Rotationsachse eingestellt werden kann. Der Kragen kann dazu vorgesehen sein, die erste Exzenterbuchse und/oder die zweite Exzenterbuchse gegeneinander oder gegen die Zentrierbuchse in der axialer Richtung zu fixieren. Beispielsweise kann der Kragen ein von dem Zentrierabschnitt in radialer Richtung vorstehender Rand sein, der in axialer Richtung als Anschlag dient.

Eine besonders einfache Ausgestaltung der ersten Exzenterbuchse und/oder der zweiten Exzenterbuchse lässt sich dadurch erreichen, dass in einer Weiterbildung die erste Exzenterbuchse und/oder die zweite Exzenterbuchse jeweils einen Hohlzylinder mit einem Ausgleichsabschnitt umfassen, wobei der Ausgleichsabschnitt sich in Richtung der jeweiligen Drehachse erstreckt und zur Veränderung des Außenumfangs des Hohlzylinders in seiner Ausdehnung (also quer zur jeweiligen Drehachse) veränderbar ist.

Der Zentrierabschnitt kann insbesondere als Hohlzylinder ausgebildet sein. Der Ausgleichsabschnitt erstreckt sich optional entlang der gesamten axialen Länge der ersten und/oder zweiten Exzenterbuchse. Der Ausgleichsabschnitt ermöglicht es, dass der Außenumfang der ersten und zweiten Exzenterbuchse veränderbar ist, insbesondere dadurch, dass sich die Ränder des Ausgleichsabschnitts zur Veränderung des Außenumfangs des Hohlzylinders aufeinander zubewegen, so dass der Umfang des Hohlzylinders verringert wird. Der Ausgleichsabschnitt kann beispielsweise aus einem elastischen Material, wie beispielsweise Gummi, hergestellt sein. Durch das Vorsehen des Ausgleichsabschnitts weist die erste und/oder zweite Exzenterbuchse einen Bereich auf, dessen Umfang veränderbar ist. Auf diese Weise lässt sich der Außenumfang der ersten und/oder zweiten Exzenterbuchse verändern. Dies ist hilfreich, um temperaturbedingte Schwankungen des Umfangs zu kompensieren.

Der Versatz kann insbesondere dadurch bereitgestellt werden, dass sich eine Dicke des Hohlzylinders in Umfangsrichtung ändert. Dieser Hohlzylinder weist abgesehen von dem Ausgleichsabschnitt eine im Querschnitt zur jeweiligen Drehachse kreisförmige Innenfläche und eine im Querschnitt zur jeweiligen Drehachse kreisförmige Außenfläche auf, deren Mittelpunkte um den jeweiligen Versatz radial versetzt sind. Damit hat der Hohlzylinder eine Wanddicke, die in der Richtung des Versatzes minimal, und auf der gegenüberliegenden Seite maximal ist. Entlang des Umfangs ändert sich die Wanddicke des Hohlzylinders von der minimalen Dicke zu der maximalen Dicke.

Eine besonders einfach herzustellende Variante des Ausgleichsabschnitts ist, wenn der Ausgleichsabschnitt einen Schlitz umfasst. Beispielsweise ist der Ausgleichsabschnitt ein sich axial erstreckender Schlitz in der ersten Exzenterbuchse und/oder der zweiten Exzenterbuchse. Schlitz ist in diesem Zusammenhang derart zu verstehen, dass die erste und/oder zweite Exzenterbuchse in Umfangsrichtung eine vollständige Unterbrechung aufweist und somit in Querschnitt in Umfangsrichtung eine Lücke vorhanden ist. Zur Veränderung des Umfangs können die dem Schlitz gegenüberliegenden Enden voneinander weg oder aufeinander zu bewegt werden.

Um die Justierung zu vereinfachen, ist es hilfreich zu wissen, in welche Richtung der erste und/oder zweite Versatz angeordnet ist. Dazu kann eine Markierung vorgesehen werden. Beispielsweise kann der Ausgleichsabschnitt als Markierung verwendet werden. In einer Weiterbildung ist als Markierung vorgesehen, dass eine gedachte Verlängerung in Richtung des ersten bzw. zweiten Versatzes den Ausgleichsabschnitt der jeweiligen Exzenterbuchse schneidet. Der Ausgleichsabschnitt ist somit die Markierung, mittels dem ein Benutzer erkennen kann, in welche Richtung der Versatz bei der ersten und/oder zweiten Exzenterbuchse liegt. Die Verlängerung kann als Gerade gedacht werden, welche die Rotationsachse mit der ersten Drehachse oder die erste Drehachse mit der zweiten Drehachse verbindet. Diese Gerade schneidet einen Umfangsbereich der ersten Exzenterbuchse und/oder der zweiten Exzenterbuchse, in welchem der Ausgleichsabschnitt, insbesondere der Schlitz, angeordnet ist. Der Ausgleichsabschnitt liegt somit, wenn die erste Exzenterbuchse und/oder die zweite Exzenterbuchse einen Hohlzylinder umfasst, im Bereich mit der dicksten Wanddicke oder der dünnsten Wanddicke.

In einer Weiterbildung ist es bevorzugt, dass der erste Versatz gleich dem zweiten Versatz ist. Insbesondere bedeutet dies, dass die Länge des ersten Versatzes gleich der Länge des zweiten Versatzes ist. Werden die erste Exzenterbuchse und die zweite Exzenterbuchse derart gedreht, dass die Richtungen der Versätze gegengleich sind, liegt die optische Achse des Okulars auf der Rotationsachse der Zentrierbuchse, da sich die beiden Versätze aufheben. Daher ist es bei dieser Ausführungsform besonders einfach möglich, einen Nullversatz bereitzustellen.

Um die aktuelle Position der optischen Achse gegenüber der Rotationsachse zu fixieren, ist es in einer Weiterbildung vorgesehen, dass ein Durchmesser der Innenfläche der Zentrierbuchse zum Fixieren mittels Kraftschluss durch die Fixiereinrichtung verringert werden kann. Die Fixiereinrichtung ist demnach in der Lage, die Zentrierbuchse radial nach innen zu verformen. Mittels der Fixiereinrichtung lässt sich somit der Umfang der Innenfläche, und damit auch der Durchmesser der Innenfläche der Zentrierbuchse, reduzieren, so dass eine radial nach Innen wirkende Kraft auf die erste Exzenterbuchse aufgebracht wird. Durch die dadurch erzeugte Reibung zwischen der Innenfläche der Zentrierbuchse und der Zentrieraußenfläche der ersten Exzenterbuchse sind diese gegeneinander fixiert. Ist auch die zweite Exzenterbuchse so angeordnet, dass sie in axialer Richtung so in die erste Exzenterbuchse eingeführt ist, dass sie auf axialer Höhe der Zentrierbuchse angeordnet ist, kann durch die Fixiereinrichtung nicht nur die relative Position der ersten Exzenterbuchse zu der Zentrierbuchse, sondern auch die relative Position der zweiten Exzenterbuchse zu der ersten Exzenterbuchse und der Zentrierbuchse fixiert werden. Dies gelingt insbesondere dann, wenn die erste Exzenterbuchse den Ausgleichsabschnitt aufweist, da dann die radiale Kraft aufgrund des veränderbaren Umfangs der ersten Exzenterbuchse besonders gut übertragen werden kann. Durch das Reduzieren des Umfangs der Innenfläche der Zentrierbuchse erhöht sich die Reibungskraft zwischen der Zentrierbuchse und der ersten Exzenterbuchse und/oder zwischen der ersten Exzenterbuchse und der zweiten Exzenterbuchse, so dass ein Drehen der ersten Exzenterbuchse gegenüber der Zentrierbuchse und/oder der zweiten Exzenterbuchse nicht länger möglich ist.

Um den Umfang der Innenfläche der Zentrierbuchse besonders einfach verringern zu können, weist die Zentrierbuchse mindestens einen entlang der Richtung der Rotationsachse verlaufenden Spalt auf. Der Spalt weist vorzugsweise die Eigenschaften des Schlitzes bei der ersten Exzenterbuchse und/oder der zweiten Exzenterbuchse auf. Beispielsweise kann die Zentrierbuchse einen Basisring und zwei, drei, vier oder mehr in Richtung der Rotationsachse von dem Basisring vorstehende Zungen aufweisen, die jeweils durch den Spalt voneinander in Umfangsrichtung getrennt sind, und deren Innenfläche die erste Exzenterbuchse um die Rotationsachse drehbar lagern.

Um den Innenumfang der ersten Zentrierbuchse besonders einfach zu verändern, ist es bevorzugt, dass die Fixiereinrichtung mindestens einen Kreiskeil, insbesondere drei Kreiskeile, und die Zentrierbuchse mindestens einen entsprechenden Kreiskeil, insbesondere drei entsprechende Kreiskeile umfasst. Mittels des Kreiskeils kann je nach Ausgestaltung ein Drehmoment in axialer und/oder radialer Richtung auf die Zentrierbuchse aufgebracht werden. Der Kreiskeil der Fixiereinrichtung steht optional in Richtung des Mittelpunkts oder der Rotationsachse der Zentrierbuchse vor und ist weiter optional ein Segment einer archimedischen Spirale. Der Kreiskeil der Zentrierbuchse ist dem Kreiskeil der Fixiereinrichtung gegenüberliegend angeordnet, beispielsweise an einer Umfangsfläche der Zentrierbuchse, insbesondere an den Zungen, und entsprechend des Kreiskeils der Fixiereinrichtung ausgebildet. Die Verwendung von drei Kreiskeilen hat sich als besonders vorteilhaft erwiesen, um eine 3K-Spannbuchse auszubilden. Durch das Drehen der Fixiereinrichtung in Richtung des Mittelpunkts der archimedischen Spirale des Kreiskeils entsteht eine Selbsthemmung zwischen der Zentrierbuchse und der Fixiereinrichtung. Die Fixiereinrichtung kann auch als Spannbuchse bezeichnet werden.

In einer anderen Variante kann die Fixiereinrichtung ein Gewinde, insbesondere ein konisches Gewinde, umfassen. In diesem Fall kann die Zentrierbuchse an ihrer Außenseite ein dazu passendes Gewinde aufweisen, so dass durch Verdrehen der Zentrierbuchse diese gegenüber der ersten Exzenterbuchse und/oder der zweiten Exzenterbuchse verklemmt bzw. verriegelt werden kann. In diesem Fall kann die Fixiereinrichtung als Überwurfmutter ausgebildet sein.

Die Erfindung schafft ferner ein Verfahren zum Zentrieren eines Okulars in einem Hauptteil eines Endoskops, wobei das Hauptteil ein proximales Ende aufweist und an dem proximalen Ende eine Zentrierbuchse mit einer Innenfläche umfasst, die eine Rotationsachse definiert. Es wird eine erste Exzenterbuchse bereitgestellt, welche in der Zentrierbuchse an dem proximalen Ende um die Rotationsachse drehbar gelagert ist und eine erste Zentrierinnenfläche aufweist, die eine erste Drehachse definiert, wobei die erste Drehachse zu der Rotationsachse um einen ersten Versatz parallel versetzt ist. Es wird ferner eine zweite Exzenterbuchse bereitgestellt, welche in der ersten Exzenterbuchse um die erste Drehachse drehbar gelagert ist und eine zweite Zentrierinnenfläche aufweist, die eine zweite Drehachse definiert, wobei die zweite Drehachse zu der ersten Drehachse um einen zweiten Versatz parallel versetzt ist. Das Okular, das eine optische Achse, die parallel zu der zweiten Drehachse ist, aufweist, wird mit der zweiten Exzenterbuchse verbunden. Das Okular wird durch Drehen der ersten Exzenterbuchse und/oder der zweiten Exzenterbuchse radial zu der Rotationsachse versetzt, bis die optische Achse mit der Rotationsachse übereinstimmt.

Die hinsichtlich des Endoskops gemachten Überlegungen, bevorzugten Ausführungsformen, Varianten und Weiterbildungen gelten für das Verfahren analog.

Die Erfindung betrifft ferner eine Okularzentriervorrichtung für ein Hauptteil eines Endoskops, wie sie oben beschrieben wurde.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Nachfolgend wird die Erfindung beispielsweise anhand der beigefügten Zeichnungen, die auch erfindungswesentliche Merkmale offenbaren, noch näher erläutert. Es zeigen:
- Fig. 1: eine schematische Darstellung eines Endoskops;
- Fig. 2: eine schematische Darstellung eines Hauptteils und einer Okularzentriervorrichtung des Endoskops von Fig. 1;
- Fig. 3: eine schematische Explosionsdarstellung des Hauptteils und der Okularzentriervorrichtung gemäß Fig. 2;
- Fig. 4: eine Querschnittsansicht einer Exzenterbuchse der Okularzentriervorrichtung von Fig. 4;
- Fig. 5: eine perspektivische Darstellung der Okularzentriervorrichtung ohne Okular von Fig. 3;
- Fig. 6: eine Querschnittsansicht und eine Seitenansicht einer Zentrierbuchse und einer Fixiereinrichtung der Okularzentriervorrichtung gemäß Fig. 5;
- Fig. 7: eine Querschnittsansicht sowie eine Schnittansicht entlang der Linie A-A der Okularzentriervorrichtung von Fig. 2; und
- Fig. 8: eine schematische Seitenansicht und eine Querschnittsansicht entlang der Linie B-B der Seitenansicht in einer im Vergleich zu Fig. 7 gezeigten weiteren Drehposition.

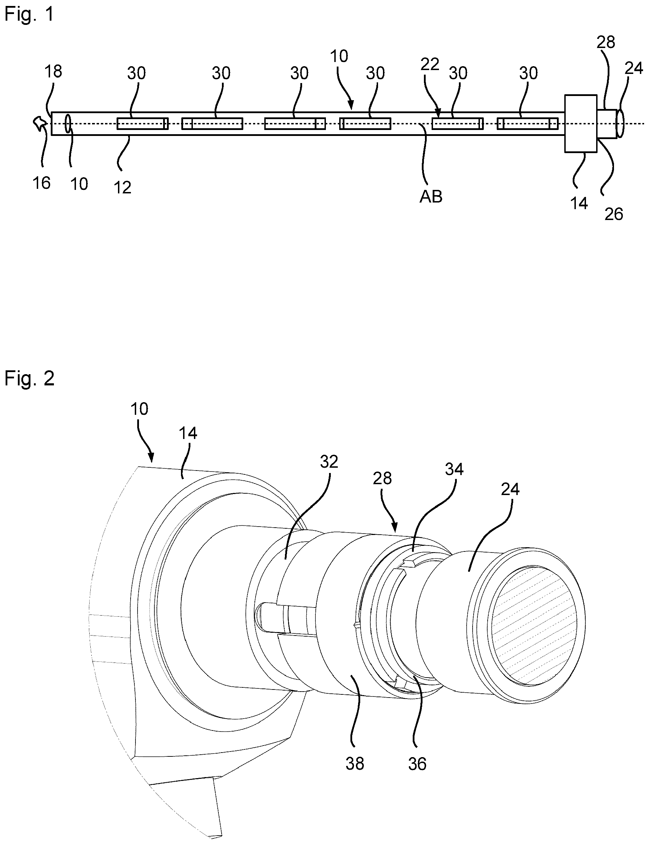

Ein erfindungsgemäßes Endoskop 10 gemäß der in Fig. 1 gezeigten Ausführungsform umfasst einen Schaft 12 und ein Hauptteil 14. Mit dem Endoskop 10 kann ein Objekt 16 beobachtet werden, welches sich beispielsweise in einer Körperöffnung befindet. Dazu wird ein distales Ende 18 des Endoskops 10, insbesondere das distale Ende 18 des Schafts 12, in die Körperöffnung eingeführt. Das Objekt 16 wird über ein Objektiv 20, das an dem distalen Ende 18 des Schafts 12 vorgesehen ist, über ein optisches Leitsystem 22 und ein Okular 24 einem Benutzer dargestellt. Das Okular 24 ist an einem proximalen Ende 26 des Hauptteils 14 vorgesehen; insbesondere ist das Okular 24 über eine erfindungsgemäße Okularzentriervorrichtung 28 an dem distalen Ende 18 des Hauptteils 14 angeordnet. Das optische Leitsystem 22 kann beispielsweise aus mehreren Linsen 30, insbesondere stabförmige Linsen 30, oder aus einem Lichtleitersystem aufgebaut sein. Das Objektiv 20 und das optische Leitsystem 22 legen eine Abbildungsachse AB fest, welche eine Mittelachse für die Abbildung des Objekts 16 darstellt.

Das Okular 24 weist eine optische Achse OA auf. Das Okular 24 kann eine oder mehrere Linsen aufweisen, die beispielsweise zueinander verschiebbar sind (z.B. entlang der optischen Achse OA), um die Abbildungseigenschaften des Okulars 24 zu verändern. Die Abbildungsachse AB wird durch das Hauptteil 14 und den Schaft 12 festgelegt. Um eine Abbildung des Objekts 16 zu ermöglichen oder zu verbessern, sollte die optische Achse OA des Okulars 24 mit der Abbildungsachse AB zusammenfallen. Zum parallelen Verschieben der optischen Achse OA des Okulars 24 ist die Okularzentriervorrichtung 28 vorgesehen. Mittels dieser lässt sich die optische Achse OA parallel in einer radialen Richtung verschieben.

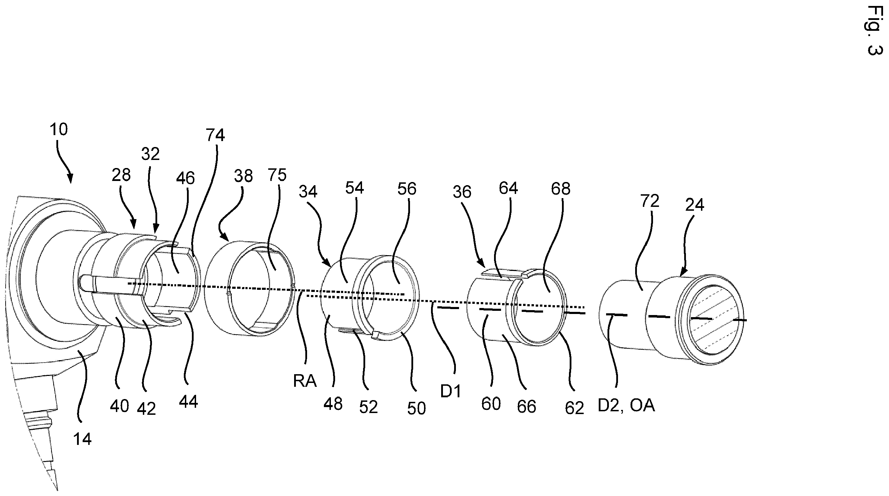

Die Okularzentriervorrichtung 28 weist eine Zentrierbuchse 32, eine erste Exzenterbuchse 34, eine zweite Exzenterbuchse 36 und eine Fixiereinrichtung 38 auf. Wie dies insbesondere in Fig. 2 und 3 zu erkennen ist, ist die Zentrierbuchse 32 mit dem Hauptteil 14 verbunden (sie kann auch einstückig mit dem Hauptteil 14 ausgebildet sein). Die Zentrierbuchse 32 weist optional einen Basisring 40 und mehrere Zungen 42 auf. Der Basisring 40 stellt die Verbindung der Zungen 42 mit dem Hauptteil 14 dar. Die Zungen 42 stehen entlang einer Rotationsachse RA der Zentrierbuchse 32 von dem Basisring 40 vor. Die Rotationsachse RA fällt beispielsweise mit der Abbildungsachse AB zusammen. Die Zungen 42 sind jeweils durch einen Spalt 44 voneinander getrennt; die Spalte 44 erstrecken sich jeweils in Richtung der Rotationsachse RA. Aufgrund der Spalte 44 lässt sich die Zentrierbuchse 32 im Bereich der Zungen 42 radial nach Innen verformen, insbesondere dadurch, dass die Zungen 42 im Vergleich zum Basisring 40 nach Innen gebogen werden.

Die Zentrierbuchse 32 weist eine Innenfläche 46 auf, die beispielsweise durch die Zungen 42 definiert ist. Ein Durchmesser der Innenfläche 46 lässt sich durch die Verformbarkeit der Zungen 42 verringern. Die Innenfläche 46 ist optional derart geformt, dass an der Innenfläche 46 ein Zylinder um die Rotationsache RA drehbar gelagert werden kann. Dazu sind beispielsweise die Zungen 42 an der Innenfläche 46 im Querschnitt senkrecht zu der Rotationsachse RA wie Kreissegmente, deren Mittelpunkt auf der Rotationsachse RA liegt, ausgebildet. Ferner können die Zungen 42 wie Segmente eines dünnwandigen Hohlzylinders ausgebildet sein, wobei die Symmetrieachse des Hohlzylinders mit der Rotationsachse RA zusammenfällt. Die Zentrierbuchse 32 weist an ihrer Außenseite drei Kreiskeile 74 auf; für jede Zunge 42 einen. Der Radius einer Außenfläche der Kreiskeile 74 nimmt in Umfangsrichtung zu, beispielsweise wie eine archimedische Spirale.

Die erste Exzenterbuchse 34 weist einen ersten Zentrierabschnitt 48 und einen ersten Kragen 50 auf. Die erste Exzenterbuchse 34 ist in der Zentrierbuchse 32 um die Rotationsachse RA drehbar gelagert. Dazu ist beispielsweise der erste Zentrierabschnitt 48 in die Zentrierbuchse 32 eingeschoben und liegt an der Innenfläche 46 an, während der erste Kragen 50 einen Anschlag in Richtung der Rotationsachse RA bildet. Die Rotationsachse RA definiert eine axiale Richtung der Okularzentriervorrichtung 28.

Die erste Exzenterbuchse 34 weist einen ersten Ausgleichsabschnitt 52 auf, der sich in Richtung der Rotationsachse RA vollständig durch die erste Exzenterbuchse 34 hindurch erstreckt. In der gezeigten Ausführungsform ist der erste Ausgleichsabschnitt 52 als Schlitz ausgebildet. Der erste Ausgleichsabschnitt 52 dient dazu, dass ein Außenumfang der ersten Exzenterbuchse 34 und damit auch ein Innenumfang der ersten Exzenterbuchse 34 durch Annähern der dem ersten Ausgleichabschnitt 52 gegenüberstehenden Enden der ersten Exzenterbuchse 34 verändert werden kann.

Die erste Exzenterbuchse 34 weist ferner eine erste Zentrieraußenfläche 54 und eine erste Zentrierinnenfläche 56 auf. Die erste Zentrieraußenfläche 54 kann beispielsweise durch eine Mantelfläche des ersten Zentrierabschnitts 48 gebildet sein. Ebenso kann die erste Zentrierinnenfläche 56 durch den ersten Zentrierabschnitt 48 gebildet sein. Die erste Zentrieraußenfläche 54 ist derart ausgebildet, dass sie in einem Hohlzylinder um die Rotationsachse RA drehbar gelagert werden kann. Dazu kann beispielsweise der erste Zentrierabschnitt 48 wie ein Hohlzylinder ausgestaltet sein.

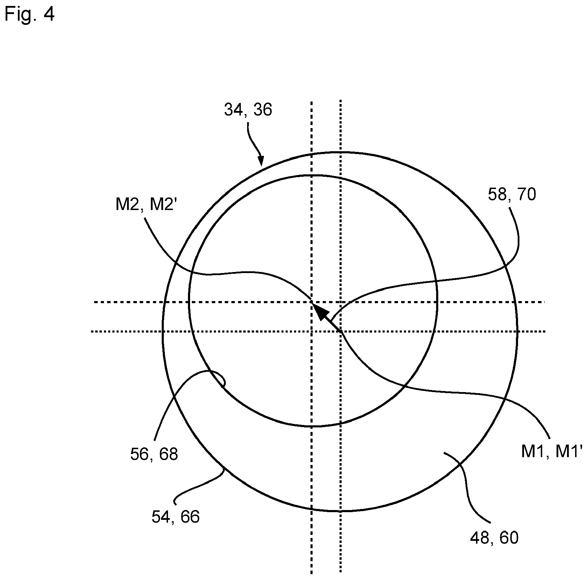

Die erste Zentrierinnenfläche 56 definiert eine erste Drehachse D1. Die erste Zentrierinnenfläche 56 ist derart ausgebildet, dass ein Zylinder um die erste Drehachse D1 drehbar gelagert werden kann. Dazu kann die erste Zentrierinnenfläche 56 wie die Innenwand eines Hohlzylinders ausgebildet sein, wobei der erste Ausgleichabschnitt 52 in Form eines Schlitzes vorgesehen ist. Wie dies insbesondere in Fig. 4 zu erkennen ist, kann die erste Zentrierinnenfläche 56 im Querschnitt wie ein Kreis ausgebildet sein, der einen ersten Mittelpunkt M1 aufweist. Die erste Zentrieraußenfläche 54 kann im Querschnitt ebenfalls wie ein Kreis ausgebildet sein, der den zweiten Mittelpunkt M2 aufweist. Der erste Mittelpunkt M1 liegt auf der Rotationsachse RA, während der zweite Mittelpunkt M2 auf der ersten Drehachse D1 liegt. Die Rotationsachse RA ist um einen ersten Versatz 58 gegenüber der ersten Drehachse D1 versetzt. Der erste Versatz 58 gibt die Richtung und die Länge des Versatzes an. Der erste Versatz 58 bestimmt also eine Exzentrizität der ersten Exzenterbuchse 34. Der erste Versatz 58 zeigt insbesondere in Richtung des ersten Ausgleichsabschnitts 52. In Fig. 4 ist der erste Versatz übertrieben groß dargestellt, wobei der erste Ausgleichsabschnitt 52 in Fig. 4 nicht dargestellt wurde.

Die zweite Exzenterbuchse 36 weist, wie z.B. in Fig. 2 ersichtlich ist, einen zweiten Zentrierabschnitt 60 und einen zweiten Kragen 62 auf. Die zweite Exzenterbuchse 36 ist in der ersten Exzenterbuchse 34 um die erste Drehachse D1 drehbar gelagert. Dazu ist beispielsweise der zweite Zentrierabschnitt 60 in die erste Exzenterbuchse 34 eingeschoben und liegt an der ersten Zentrierinnenfläche 56 an, während der zweite Kragen 62 einen Anschlag in Richtung der ersten Drehachse D1 bildet.

Die zweite Exzenterbuchse 36 weist einen zweiten Ausgleichsabschnitt 64 auf, der sich in Richtung einer zweiten Drehachse D2 der zweiten Exzenterbuchse 36 vollständig durch die zweite Exzenterbuchse 36 hindurch erstreckt. In der gezeigten Ausführungsform ist der zweite Ausgleichsabschnitt 64 als Schlitz ausgebildet. Der zweite Ausgleichsabschnitt 64 dient dazu, dass durch Annähern der dem zweiten Ausgleichabschnitt 64 gegenüberstehenden Enden der zweiten Exzenterbuchse 36 ein Außenumfang der zweiten Exzenterbuchse 36 und damit auch ein Innenumfang der zweiten Exzenterbuchse 36 verändert, insbesondere verringert, werden kann.

Die zweite Exzenterbuchse 36 weist ferner eine zweite Zentrieraußenfläche 66 und eine zweite Zentrierinnenfläche 68 auf. Die zweite Zentrieraußenfläche 66 kann beispielsweise durch eine Mantelfläche des zweiten Zentrierabschnitts 60 gebildet sein. Ebenso kann die zweite Zentrierinnenfläche 68 durch den zweiten Zentrierabschnitt 60 gebildet sein. Die zweite Zentrieraußenfläche 66 ist derart ausgebildet, dass sie in einem Hohlzylinder um die erste Drehachse D1 drehbar gelagert werden kann. Dazu kann beispielsweise der zweite Zentrierabschnitt 60 wie ein Hohlzylinder ausgestaltet sein.

Die zweite Zentrierinnenfläche 68 definiert die zweite Drehachse D2. Die zweite Zentrierinnenfläche 68 ist derart ausgebildet, dass ein Zylinder um die zweite Drehachse D2 drehbar gelagert werden kann. Die zweite Drehachse D2 ist parallel zu der ersten Drehachse D1 und der Rotationsachse RA angeordnet. Dazu kann die zweite Zentrierinnenfläche 68 wie die Innenwand eines Hohlzylinders ausgebildet sein, wobei der zweite Ausgleichabschnitt 64 in Form eines Schlitzes vorgesehen ist. Die zweite Zentrierinnenfläche 68 ist im Querschnitt analog wie die in Fig. 4 dargestellte erste Zentrierinnenfläche 56 ausgestaltet. Die zweite Zentrierinnenfläche 68 kann im Querschnitt wie ein Kreis ausgebildet sein, der einen ersten Mittelpunkt M1' aufweist. Die zweite Zentrieraußenfläche 66 kann im Querschnitt ebenfalls wie ein Kreis ausgebildet sein, der den zweiten Mittelpunkt M2' aufweist. Der erste Mittelpunkt M1' liegt auf der ersten Drehachse D1, während der zweite Mittelpunkt M2' auf der zweiten Drehachse D2 liegt. Die zweite Drehachse D2 ist um einen zweiten Versatz 70 gegenüber der ersten Drehachse D1 versetzt. Der zweite Versatz 70 gibt die Richtung und die Länge des Versatzes an. Der zweite Versatz 70 bestimmt also eine Exzentrizität der zweiten Exzenterbuchse 36.

Der erste Versatz 58 und der zweite Versatz 70 können identisch ausgebildet sein, insbesondere sind die Längen des ersten und zweiten Versatzes 58, 70 identisch.

Das Okular 24 ist mit der zweiten Exzenterbuchse 36 verbunden. Dazu kann beispielsweise das Okular 24 einen Einschubabschnitt 72 aufweisen, der in die zweite Exzenterbuchse 36 eingeschoben ist. Die optische Achse OA des Okulars 24 fällt mit der zweiten Drehachse D2 zusammen.

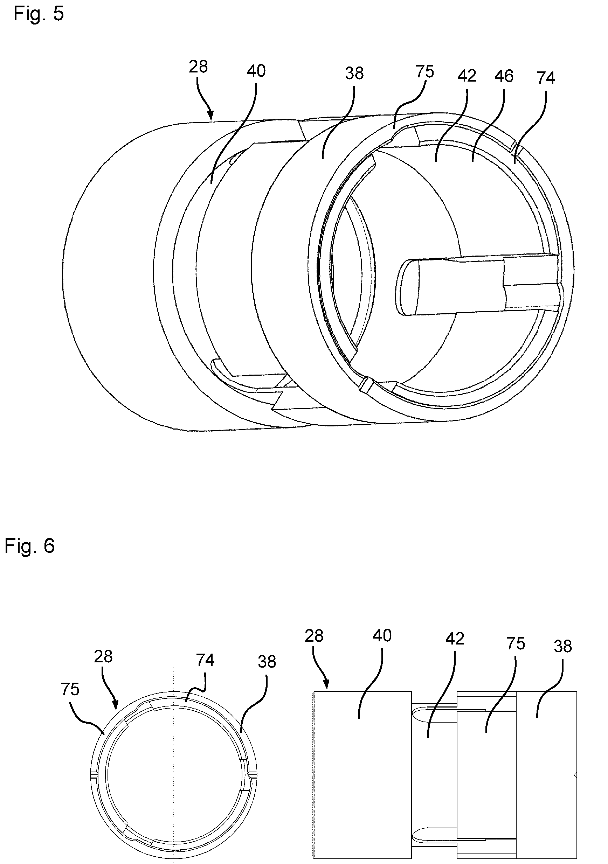

Zur Fixierung des Okulars 24 in der zweiten Exzenterbuchse 36, der zweiten Exzenterbuchse 36 in der ersten Exzenterbuchse 34 und der ersten Exzenterbuchse 34 in der Zentrierbuchse 32 ist die Fixiereinrichtung 38 vorgesehen. Diese befindet sich an einer Außenfläche der Zentrierbuchse 32 und dient dazu, einen Durchmesser der Innenfläche 46 der Zentrierbuchse 32, insbesondere der Zungen 42, zu verkleinern. Dazu weist die Fixiereinrichtung 38 an ihrer Innenseite wenigstens einen Kreiskeil 75, insbesondere drei Kreiskeile 75, auf. Die Kreiskeile 75 sind wie Segmente einer archimedischen Spirale angeordnet, wie dies in Fig. 5 und 6 zu erkennen ist, und insbesondere gleichmäßig um den Umfang der Fixiereinrichtung 38 ausgebildet. Durch Drehen der Fixiereinrichtung 38 gegenüber der Zentrierbuchse 32 entsteht aufgrund der Kreiskeile 74 und 75 ein Drehmoment, das axial und radial auf die Zentrierbuchse 32 wirkt. Die Zentrierbuchse 32, insbesondere deren Zunge 42, wird radial nach innen verformt, so dass es aufgrund der dadurch bewirkten Verringerung des Durchmessers der Innenfläche 46 an den Stellen, an denen sich die Kreiskeile 74, 75 berühren, zu einem Kraftschluss zwischen der Zentrierbuchse 32 und ersten Exzenterbuchse 34 kommt. Da in der ersten Exzenterbuchse 34 der erste Ausgleichsabschnitt 52 vorgesehen ist, lässt sich durch das Verdrehen der Fixiereinrichtung 38 erzeugte Drehmoment von der Zentrierbuchse 32 auf die erste Exzenterbuchse 34 und damit auch auf die zweite Exzenterbuchse 36 übertragen. Der Umfang der ersten Exzenterbuchse 34 ist somit veränderbar, wodurch es zu einem Kraftschluss zwischen der ersten Exzenterbuchse 34 und der zweiten Exzenterbuchse 36 kommt. Da auch der Außenumfang der zweiten Exzenterbuchse 36 durch den zweiten Ausgleichsabschnitt 64 veränderbar ist, kommt es darüber hinaus zu einem Kraftschluss zwischen der zweiten Exzenterbuchse 36 und dem Okular 24. Das Okular 24 ist somit gegenüber der Zentrierbuchse 32 fixiert.

Die Fixiereinrichtung 38 kann jedoch auch als eine Mutter ausgebildet sein, die mittels eines Gewindes auf die Zentrierbuchse 32 aufgeschraubt werden kann.

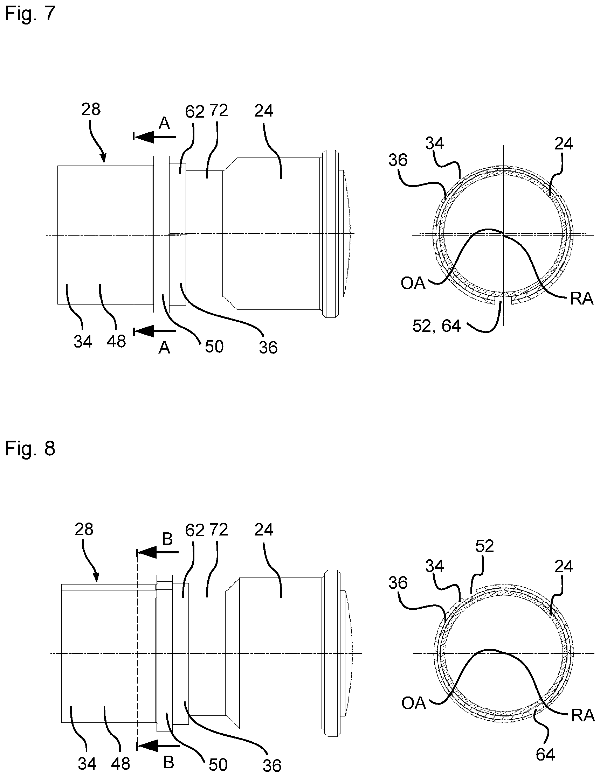

Um die optische Achse OA des Okulars 24 gegenüber der Abbildungsachse AB oder der Rotationsachse RA zu verändern, werden die erste Exzenterbuchse 34 und/oder die zweite Exzenterbuchse 36 entlang ihres Umfangs gedreht. Aufgrund des ersten Versatzes 58 und des zweiten Versatzes 70 lässt sich die optische Achse OA parallel gegenüber der Rotationsachse RA versetzen, wie dies in Fig. 7 gezeigt ist. Das Verdrehen der ersten und/oder zweiten Exzenterbuchse 34, 36 führt dazu, dass die jeweilige Drehachse D1, D2 gegenüber der jeweiligen Zentrieraußenfläche 54, 66 in Richtung des Versatzes verschoben wird. Wird der erste Versatz 58 gegengleich zu dem zweiten Versatz 70 angeordnet, so befindet sich die Rotationsachse RA identisch zu der optischen Achse OA, wie dies in Fig. 8 gezeigt ist.

Die Lage des ersten Versatzes 58 und/oder des zweiten Versatzes 70 lässt sich optional durch Markierungen erkennen. Ein Beispiel für eine solche Markierung ist der erste Ausgleichsabschnitt 52 und/oder der zweite Ausgleichsabschnitt 64. Diese werden als Markierung so angeordnet, dass eine gedachte Verlängerung des ersten Versatzes 58 den ersten Ausgleichsabschnitt 52 und/oder des zweiten Versatzes 70 den zweiten Ausgleichsabschnitt 64 schneidet. Auf diese Weise gibt die Lage des ersten Ausgleichsabschnitts 52 und/oder des zweiten Ausgleichsabschnitts 64 die Richtung des jeweiligen Versatzes 58, 70 an.

## Patentansprüche

1. Endoskop, umfassend
- ein ein proximales Ende (26) aufweisendes Hauptteil (14), welches an dem proximale Ende (26) eine Zentrierbuchse (32) mit einer Innenfläche (46), die eine Rotationsachse (RA) definiert, umfasst,
- eine erste Exzenterbuchse (34), welche in der Zentrierbuchse (32) an dem proximalen Ende (26) um die Rotationsachse (RA) drehbar gelagert ist und eine erste Zentrierinnenfläche (56) aufweist, die eine erste Drehachse (D1) definiert, wobei die erste Drehachse (D1) zu der Rotationsachse (RA) um einen ersten Versatz (58) parallel versetzt ist,
- eine zweite Exzenterbuchse (36), welche in der ersten Exzenterbuchse (34) um die erste Drehachse (D1) drehbar gelagert ist und eine zweite Zentrierinnenfläche (68) aufweist, die eine zweite Drehachse (D2) definiert, wobei die zweite Drehachse (D2) zu der ersten Drehachse (D1) um einen zweiten Versatz (70) parallel versetzt ist, und
- ein Okular (24), welches mit der zweiten Exzenterbuchse (36) verbunden ist und eine optische Achse (OA) aufweist, die parallel zu der zweiten Drehachse (D2) ist,
- wobei das Okular (24) durch Drehen der ersten Exzenterbuchse (34) und/oder der zweiten Exzenterbuchse (36) radial zur Rotationsachse (RA) versetzbar ist.

2. Endoskop nach Anspruch 1, **dadurch gekennzeichnet, dass** die erste Exzenterbuchse (34) und/oder die zweite Exzenterbuchse (36) jeweils einen Hohlzylinder mit einem Ausgleichsabschnitt (52, 64) umfassen, wobei der Ausgleichsabschnitt (52, 64) sich in Richtung der jeweiligen Drehachse (D1, D2) erstreckt und zur Veränderung eines Außenumfangs des Hohlzylinders in seiner Ausdehnung veränderbar ist.

3. Endoskop nach Anspruch 2, **dadurch gekennzeichnet, dass** der Ausgleichsabschnitt (52, 64) einen Schlitz umfasst.

4. Endoskop nach Anspruch 2 oder 3, **dadurch gekennzeichnet, dass** eine gedachte Verlängerung in Richtung des ersten Versatzes (58) den Ausgleichsabschnitt (52) der ersten Exzenterbuchse (34) schneidet und/oder dass eine gedachte Verlängerung des zweiten Versatzes (70) den Ausgleichsabschnitt (64) der zweiten Exzenterbuchse (36) schneidet.

5. Endoskop nach einem der obigen Ansprüche, **dadurch gekennzeichnet, dass** eine Länge des ersten Versatzes (58) gleich einer Länge des zweiten Versatzes (70) ist.

6. Endoskop nach einem der obigen Ansprüche, **dadurch gekennzeichnet, dass** ein Durchmesser der Innenfläche (46) der Zentrierbuchse (32) zum Fixieren mittels Kraftschluss durch eine Fixiereinrichtung (38) verringert werden kann.

7. Endoskop nach Anspruch 6, **dadurch gekennzeichnet, dass** die Zentrierbuchse (32) mindestens einen Spalt (44) aufweist.

8. Endoskop nach Anspruch 6 oder 7, **dadurch gekennzeichnet, dass** die Fixiereinrichtung (38) mindestens einen Kreiskeil (75), insbesondere drei Kreiskeile (75), und die Zentrierbuchse (32) mindestens einen entsprechenden Kreiskeil (74), insbesondere drei entsprechende Kreiskeile (74), umfasst.

9. Endoskop nach Anspruch 6 oder 7, **dadurch gekennzeichnet, dass** die Fixiereinrichtung (38) ein Gewinde, insbesondere konisches Gewinde, umfasst.

10. Verfahren zum Zentrieren eines Okulars (24) in einem Hauptteil (14) eines Endoskops (10),
- wobei das Hauptteil (14) ein proximales Ende (26) aufweist und an dem proximale Ende (26) eine Zentrierbuchse (32) mit einer Innenfläche (46), die eine Rotationsachse (RA) definiert, umfasst,
- wobei eine erste Exzenterbuchse (34) bereitgestellt wird, welche in der Zentrierbuchse (32) an dem proximalen Ende (26) um die Rotationsachse (RA) drehbar gelagert ist und eine erste Zentrierinnenfläche (56) aufweist, die eine erste Drehachse (D1) definiert, wobei die erste Drehachse (D1) zu der Rotationsachse (RA) um einen ersten Versatz (58) parallel versetzt ist,
- wobei eine zweite Exzenterbuchse (36) bereitgestellt wird, welche in der ersten Exzenterbuchse (34) um die erste Drehachse (D1) drehbar gelagert ist und eine zweite Zentrierinnenfläche (68) aufweist, die eine zweite Drehachse (D2) definiert, wobei die zweite Drehachse (D2) zu der ersten Drehachse (D1) um einen zweiten Versatz (70) parallel versetzt ist,
- wobei das Okular (24), das eine optische Achse (OA), die parallel der zweiten Drehachse (D2) ist, aufweist, mit der zweiten Exzenterbuchse (36) verbunden wird und
- wobei das Okular (24) durch Drehen der ersten Exzenterbuchse (34) und/oder der zweiten Exzenterbuchse (36) radial zu der Rotationsachse (RA) versetzt wird, bis die optische Achse (OA) mit der Rotationsachse (RA) übereinstimmt.
